# EUROPÄISCHE PATENTSCHRIFT

(11) **EP 0 452 834 B1**
(45) Veröffentlichungstag und Bekanntmachung des Hinweises auf die Patenterteilung: **12.07.1995**
(21) Anmeldenummer: 91105910.3
(22) Anmeldetag: 13.04.1991
(51) Int. Cl.: A61B 5/0416, H01R 9/07

(54) **Flexible Flächenelektrode**
Flexible sheet electrode
Electrode flexible en forme de feuille

(30) Priorität: 20.04.1990 AT 922/90
(43) Veröffentlichungstag der Anmeldung: 23.10.1991
(73) Patentinhaber: D. Swarovski & Co., A-6112 Wattens (AT)
(72) Erfinder: Andreas, Kurtze, A-6122 Fritzens (AT)
(74) Vertreter: Hofinger, Engelbert, DDr.

(56) Entgegenhaltungen:
- EP-A- 0 268 364
- WO-A-81/01646
- CH-A- 512 833
- FR-A- 2 193 571
- US-A- 3 154 365

## Beschreibung

Die Erfindung bezieht sich auf eine flexible Flächenelektrode zum Zu- und Ableiten von Strömen zu bzw. von der Haut eines menschlichen Körpers in Form eines isolierenden Trägers, der mit mindestens einer elektrisch leitenden Folie belegt ist, welche sich in eine von der Flächenelektrode abstehende Lasche fortsetzt, wobei die Lasche derart um einen versteifenden plattenförmigen Formteil herumgebogen ist, daß die elektrisch leitenden Folien nach außen weisen.

Bei Flächenelektroden, wie sie insbesondere als Neutralelektroden in der Chirurgie Anwendung finden, besteht der Wunsch nach einer rasch herstellbaren und dennoch sicheren Verbindung mit dem der Elektrode zugeordneten Steckverbinder. Dabei hat es sich als nachteilig erwiesen, wenn die Kontaktlasche der Flächenelektrode genauso flexibel ist wie die Elektrode selbst, da sie sich beim Einschieben in eine Kontakthülse oder beim Verbinden mit einer Anschlußklammer (DE-A-37 39 516) leicht verbiegt.

Aus DE-A-35 44 483 ist zwar bereits eine flexible Flächenelektrode bekannt geworden, bei der die leitende Fläche der Lasche um den Stirnrand eines Formteiles herumgebogen und dadurch versteift ist. Die Kontaktierung der Lasche erfolgt jedoch nicht im Bereich des Stirnrandes sondern im parallel zur Hülse verlaufenden Endbereich der Lasche. Der leitende Teil der Lasche wird dabei mittels eines federbelasteten Anpreßteiles in kraftschlüssigen Kontakt mit leitfähigen Belägen gebracht, welche an der Wand der Hülse angebracht sind. Dieser federbelastete Anpreßteil vermehrt die Bauhöhe des Steckverbinders ganz erheblich. Nachteilig am Steckverbinder gemäß DE-A-35 44 483 ist außerdem, daß die Endstellung der Lasche in der Hülse nicht hinreichend genau definiert ist. Bei teilweise herausgezogener Lasche besteht immer noch ein elektrischer Kontakt. Das bedeutet, daß der leitende Teil der Laschenoberfläche unmittelbar mit der Haut des Patienten in Berührung kommen kann. Zur Berührung mit der Haut ist jedoch nur ein Teil der Elektrode geeignet, dessen elektrischer Widerstand wesentlich höher ist als jener der Kontaktflächen der Lasche.

Die Erfindung vermeidet dieses Problem dadurch, daß das Ende der Lasche zwischen dem Formteil und einer Klemmplatte festgehalten ist, daß die elektrisch leitenden Folien am Stirnrand des Formteiles zur Kontaktierung freiliegen, daß der zwischen dem Stirnrand des Formteiles und der eigentlichen Flächenelektrode verlaufende Teil der Lasche durch eine Abdeckplatte abgedeckt ist und daß die Teile Formteil, Klemmplatte und Abdeckplatte durch Filmscharniere verbunden sind.

Dadurch daß erfindungsgemäß die vom Stirnrand des Formteiles entfernten Teile der Kontaktlasche abgedeckt sind, ist es ungefährlich, wenn die Kontaktlasche etwas aus dem korrespondierenden Steckverbinder herausgezogen ist. Allerdings wird bei der erfindungsgemäßen Konstruktion der elektrische Kontakt auch bei geringfügigem Herausziehen der Lasche bereits unterbrochen, weshalb es günstig ist, wenn am Formteil der Klemmplatte oder der Abdeckplatte Ausnehmungen oder Vorsprünge zur formschlüssigen Verbindung mit korrespondierenden Vorsprüngen bzw. Ausnehmungen am Steckverbinder vorgesehen sind. Die Erfindung ermöglicht eine flache Bauweise des Steckverbinders, wodurch die schmerzenden Druckstellen vermieden werden, welche entstehen, wenn der Patient auf herkömmlichen Einrichtungen zu liegen kommt.

Aus der US-PS 3,154,365 ist ein Stecker bekannt, bei dem ein Leiterband um einen plattenförmigen Formteil derart herumgebogen ist, daß die elektrischen Leiterbahnen am Stirnrand zur Kontaktierung freiliegen. Das Leiterband wird in dieser Position fixiert, indem der Formteil durch die Öffnung eines Klemmteiles geschoben wird und im Klemmteil fixiert wird.

Für die Ausbildung des die Laschen versteifenden Formteiles sind verschiedene konstruktive Lösungen denkbar. Die Filmscharniere zum Verbinden des Formteils, der Klemmplatte und der Abdeckplatte können unterschiedlich angeordnet sein, wie anschließend anhand der Zeichnung erläutert wird.

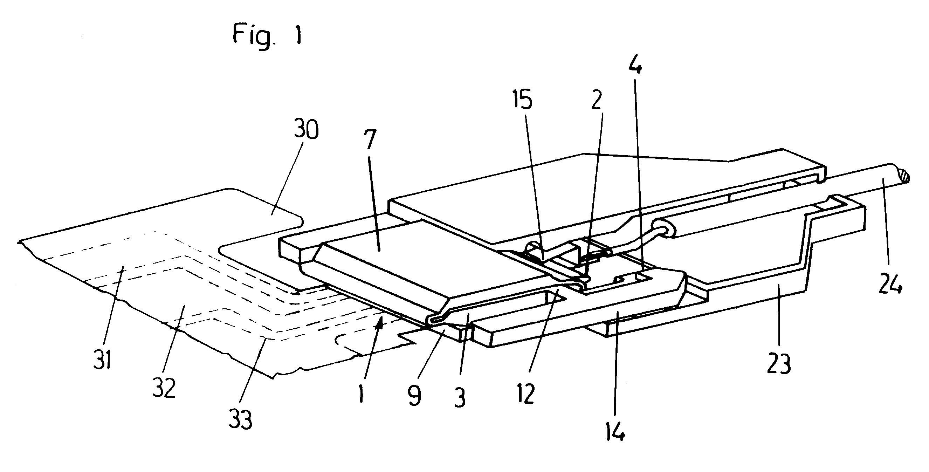

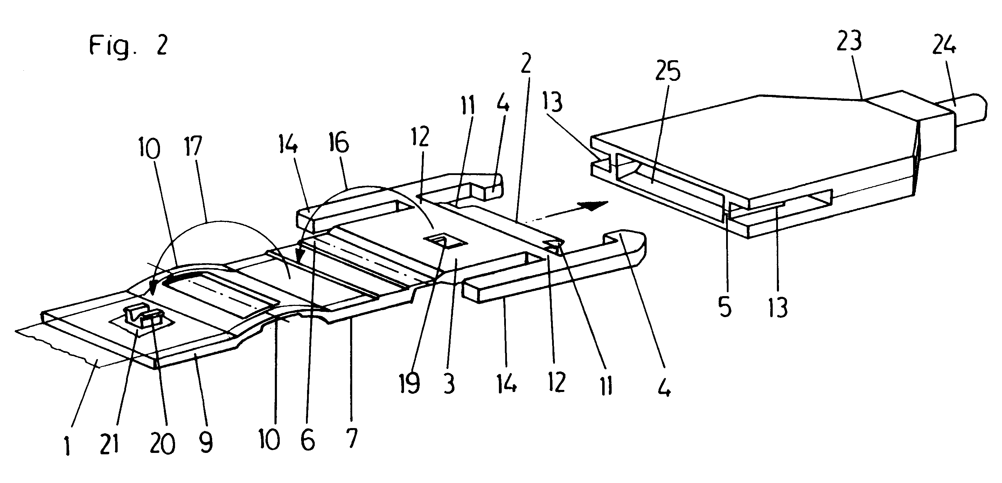

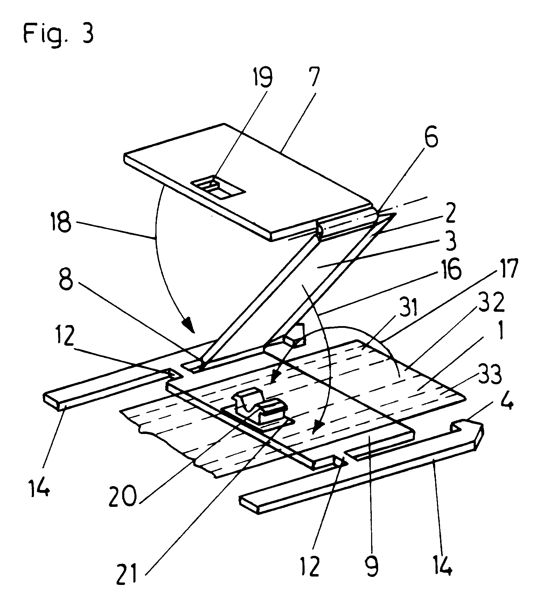

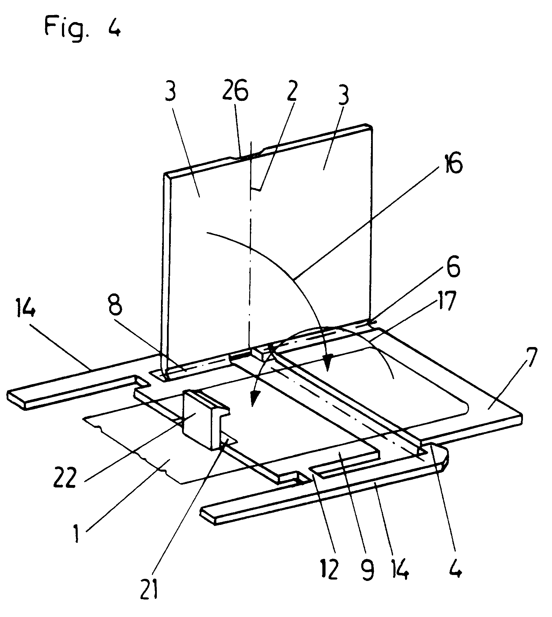

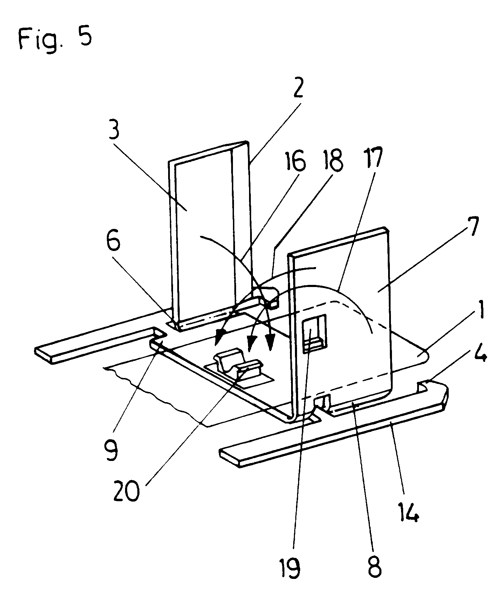

Fig. 1 zeigt den erfindungswesentlichen Teil einer Flächenelektrode in Verbindung mit einem teilweise geschnittenen Steckverbinder, Fig. 2 zeigt die in Fig. 1 dargestellten Teile getrennt vor der Befestigung der Lasche 1 am Formteil 3, Fig. 3 ist ein zweites Ausführungsbeispiel für die Verbindung von Lasche 1 und Formteil 3, Fig. 4 und 5 weitere Ausführungsbeispiele hiefür.

Der in Fig. 1 dargestellte Steckverbinder 23 dient dazu, über ein Kabel 24 und eine Federklemme 15 den Kontakt mit der nur teilweise dargestellten Flächenelektrode herzustellen. Diese besteht im wesentlichen aus einem mit der Haut des Patienten zu verklebenden Träger 30, an dessen Unterseite leitende Folien 31 - 33 angebracht sind, welche sich in die Lasche 1 fortsetzen. Die Flächenelektrode ist aber mit mindestens einer Lasche 1 versehen, welche an ihrer Unterseite leitet. Zur Versteifung der Lasche 1 dient ein Formteil 3, um dessen Stirnrand 2 die Lasche 1 herumgebogen ist. Die Kontaktierung der Lasche 1 erfolgt im Bereich dieses Stirnrandes 2.

Um die Lasche 1 formschlüssig im Steckverbinder 23 zu sichern, sind an der Außenseite der Hülse 5 des Steckverbinders 23 Ausnehmungen 13 vorgesehen. In diese Ausnehmungen greifen korrespondierende Vorsprünge 4, welche durch Zusammendrücken der Arme 14 aus den Ausnehmungen 13 entfernt werden können. Dabei erfolgt eine elastische Verformung der Stege 12, welche die Arme 14 mit dem Formteil 3 verbinden. Die Stege 12 bilden beim Einschieben des Formteiles 3 in die Öffnung 25 der Hülse 5 einen Anschlag, welcher die Endlage der Lasche 1 genau festlegt. In dieser Endlage liegt die Federklemme 15, die sich maulartig nach außen öffnet, im Bereich des Stirnrandes 2 des Formteiles 3 federnd an der Lasche 1 an.

Fig. 2 zeigt, wie die Lasche 1 mit dem Formteil 3 nach Fig. 1 verbunden wird. Zunächst wird die Lasche 1 mit der leitenden Seite nach unten auf jene Teile aufgelegt, welche schließlich als Klemmplatte 7 und Abdeckplatte 9 fungieren sollen. Dabei durchdringt ein Druckknopf 20 eine Ausnehmung 21 in der mit ihrer leitenden Seite nach unten weisenden Lasche 1. Zunächst wird nun der Formteil 3 um das Filmscharnier 6 in Richtung des Pfeiles 16 umgeklappt, wodurch der freie Rand der Lasche 1 von der Klemmplatte 7 gegen den Formteil 3 gedrückt wird. Ein weiteres Umklappen der beiden aneinander liegenden Teile 3 und 7 um die Längsmitte der Bügel 10 führt zu der in Fig. 1 dargestellten Situation, in welcher der Formteil 3 mit der Abdeckplatte 9, welche die leitende Unterseite der Lasche 1 schützt, verbunden ist, da der Druckknopf 20 in die Rastöffnung 19 des Formteiles 3 eingerastet ist. Die Bügel 10 liegen nunmehr in Einkerbungen 11 des Stirnrandes 2 des Formteiles 3. Die leitende Unterseite der Lasche 1 umgibt den schneidenartig zusammenlaufenden Stirnrand 2 des Formteiles 3, sodaß hier ein Kontakt mit den Federklemmen 15 des Steckverbinders 23 erfolgen kann. Wenn die Folien 31 - 33 nicht untereinander verbunden sind, muß natürlich für jede derselben eine Federklemme 15 vorgesehen sein.

Das Ausführungsbeispiel nach Fig. 3 unterscheidet sich von jenem nach Fig. 1 und 2 nur in der Art der Befestigung von Lasche 1 und Formteil 3. Sowohl das Filmscharnier 6, das den Formteil 3 mit der Klemmplatte 7 verbindet, wie das Filmscharnier 8 zur Verbindung von Formteil 3 und Abdeckplatte 9 verlaufen hier parallel zur Lasche 1. Die Verbindung der Lasche 1 mit dem Formteil 3 geht nun so vor sich, daß zunächst um das Filmscharnier 8 eine Verschwenkung des Formteiles 3 im Sinne des Pfeiles 16 erfolgt. Anschließend wird das freie Ende der Lasche 1 um den Stirnrand 2 des Formteiles 3 im Sinne des Pfeiles 17 gebogen und anschließend die Klemmplatte 7 im Sinne des Pfeiles 18 um das Filmscharnier 6 geschwenkt, um das freie Ende der Lasche 1 auf dem Formteil 3 festzuhalten. Das aus Formteil 3, Klemmplatte 7 und Abdeckplatte 9 bestehende Paket wird durch eine Rastverbindung fixiert, welche durch den Druckknopf 20 und die Rastöffnung 19 gebildet ist.

Die Arme 14 sind beim Ausführungsbeispiel nach Fig. 3 mittels der Stege 12 an der Abdeckplatte 9 befestigt. Sie könnten jedoch auch, wie dies in Fig. 2 dargestellt ist, am Formteil 3 angeschlossen sein. Dies würde es ermöglichen, den in Fig. 1 und 2 dargestellten Steckverbinder 23 wahlweise im Zusammenhang mit der versteiften Lasche gemäß Fig. 1 oder 3 zu verwenden.

Beim Ausführungsbeispiel nach Fig. 4 wird wiederum die Lasche 1 mit ihrer nach unten weisenden, leitenden Seite auf die Klemmplatte 7 und die Abdeckplatte 9 gelegt, wobei eine Klammer 22 die Öffnung 21 in der Lasche 1 durchdringt. Daraufhin wird der Formteil 3 um die Filmscharniere 6 und 8 im Sinne des Pfeiles 16 umgeklappt und dabei einerseits mit der Klemmplatte 7 und andererseits mit der Abdeckplatte 9 verbunden. Der erfindungswesentliche Stirnrand 2 des Formteiles 3 entsteht jedoch erst durch Falten des Formteiles 3 im Sinne des Pfeiles 17, was durch eine Kerbe 26 im Formteil 3 ermöglicht wird. Die Fixierung des nunmehr aus vier Lagen bestehenden Pakets erfolgt durch die Klammer 22. Bei der Ausbildung des zugehörigen Steckverbinders 23 ist darauf zu achten, daß die Vorsprünge 4 bei dieser Ausführung gegenüber dem Stirnrand 2 des Formteiles 3 nach unten versetzt sind und daher auch die Ausnehmungen 13 am Steckverbinder 23 gegenüber der Ausführung nach Fig. 1 versetzt sind.

Beim Ausführungsbeispiel nach Fig. 5 wird die Lasche 1 zunächst ebenso wie etwa beim Ausführungsbeispiel nach Fig. 3 auf die Abdeckplatte 9 gelegt, anschließend wird der Formteil 3 im Sinne des Pfeiles 16 um das Filmscharnier 6 umgeklappt und die Lasche 1 um den Stirnrand 2 des Formteiles 3 im Sinne des Pfeiles 17 verschwenkt. Die Fixierung der Lasche 1 erfolgt durch Niederklappen der Klemmplatte 7 im Sinne des Pfeiles 18 und durch Verankerung des Druckknopfes 20 in der Rastöffnung 19.

Allen dargestellten Ausführungsbeispielen sind eine Reihe von Vorteilen gemeinsam:
Dadurch, daß die Arme 14 an der Außenseite des Steckverbinders anliegen, ist der Kontaktraum praktisch flüssigkeitsdicht nach außen abgeschlossen. Die beidseitige Anordnung der Arme 14 verhindert ein unabsichtliches Lösen der Steckverbindung.

Je mehr Kontakte benötigt werden, umso mehr kommt der Vorteil der stirnseitigen Kontaktierung der Lasche gegenüber dem Stand der Technik mit seitlich angeordneten Kontakten zur Geltung.

Die kabelseitige Anschlußbuchse kann, soweit die Anschlüsse nicht geräteseitig verpolungssicher sein müssen, beim gleichen Handhabungsaufwand symmetrisch benutzbar ausgeführt werden, wodurch die Anwendung vereinfacht wird.

## Patentansprüche

1. Flexible Flächenelektrode zum Zu- und Ableiten von Strömen zu bzw. von der Haut eines menschlichen Körpers in Form eines isolierenden Trägers (30), der mit mindestens einer elektrisch leitenden Folie (31 - 33) belegt ist, welche sich in eine von der Flächenelektrode abstehende Lasche (1) fortsetzt, wobei die Lasche (1) derart um einen versteifenden plattenförmigen Formteil (3) herumgebogen ist, daß die elektrisch leitenden Folien (31 - 33) nach außen weisen, dadurch gekennzeichnet, daß
- das Ende der Lasche (1) zwischen dem Formteil (3) und einer Klemmplatte (7) festgehalten ist,
- die elektrisch leitenden Folien (31 - 33) am Stirnrand des Formteiles (3) zur Kontaktierung freiliegen
- der zwischen dem Stirnrand (2) des Formteiles (3) und der eigentlichen Flächenelektrode verlaufende Teil der Lasche (1) durch eine Abdeckplatte (9) abgedeckt ist, und
- daß die Teile Formteil (3), Klemmplatte (7) und Abdeckplatte (9) durch Filmscharniere verbunden sind.

2. Flächenelektrode nach Anspruch 1, dadurch gekennzeichnet, daß die Filmscharniere zwischen Formteil (3) und Klemmplatte (7), sowie zwischen Klemmplatte (7) und Abdeckplatte (9) angebracht sind.

3. Flächenelektrode nach Anspruch 2, dadurch gekennzeichnet, daß die Filmscharniere quer zur Lasche (1) verlaufen.

4. Flächenelektrode nach Anspruch 2 oder 3, dadurch gekennzeichnet, daß zwischen Klemmplatte (7) und Abdeckplatte (9) Bügel (10) in Längsrichtung der Lasche (1) verlaufen, welche die Klemmplatte (7) mit der Abdeckplatte (9) verbindende Filmscharniere aufweisen.

5. Flächenelektrode nach Anspruch 4, dadurch gekennzeichnet, daß der Rand (2) des Formteiles (3) Einkerbungen (11) zur Aufnahme der Bügel (10) aufweist.

6. Flächenelektrode nach Anspruch 1, dadurch gekennzeichnet, daß die Filmscharniere (6,8) zwischen Klemmplatte (7) und Formteil (3) sowie zwischen Formteil (3) und Abdeckplatte (9) angebracht sind.

7. Flächenelektrode nach Anspruch 6, dadurch gekennzeichnet, daß die Filmscharniere (6,8) in der Längsrichtung der Lasche verlaufen.

8. Flächenelektrode nach Anspruch 1, dadurch gekennzeichnet, daß das Formteil (3) durch in Längsrichtung der Lasche (1) verlaufende Filmscharniere (6,8) sowohl mit der Abdeckplatte (9) als auch mit der Klemmplatte (7) verbunden ist, wobei das Formteil (3) eine senkrecht zu den Filmscharnieren (6,8) verlaufende Kerbe (26) zum Falten des Formteiles (3) aufweist.

9. Flächenelektrode nach einem der Ansprüche 1 bis 8, dadurch gekennzeichnet, daß die jeweils außen liegenden Teile des aus Formteil (3), Klemmplatte (7) und Abdeckplatte (9) bestehenden Stapels durch eine Schnappverbindung verbunden sind.

## Claims

1. A flexible flat electrode for carrying currents to and from the skin of a human body in the form of an insulating carrier (30) which is covered with at least one electrically conductive foil (31-33) which is extended into a tongue (1) projecting from the flat electrode, wherein the tongue (1) is bent around a stiffening plate-like shaped portion (3) in such a way that the electrically conductive foils (31-33) face outwardly, characterised in that
- the end of the tongue (1) is held fast between the shaped portion (3) and a clamping plate (7),
- the electrically conductive foils (31-33) are exposed at the end edge of the shaped portion (3) for contacting purposes,
- the part of the tongue (1) which extends between the end edge (2) of the shaped portion (3) and the actual flat electrode is covered by a cover plate (9), and
- the parts consisting of the shaped portion (3), the clamping plate (7) and the cover plate (9) are joined by film hinges.

2. A flat electrode according to claim 1 characterised in that the film hinges are disposed between the shaped portion (3) and the clamping plate (7), and between the clamping plate (7) and the cover plate (9).

3. A flat electrode according to claim 2 characterised in that the film hinges extend transversely to the tongue (1).

4. A flat electrode according to claim 2 or claim 3 characterised in that loop portions (10) extend in the longitudinal direction of the tongue (1) between the clamping plate (7) and the cover plate (9), the loop portions having film hinges connecting the clamping plate (7) to the cover plate (9).

5. A flat electrode according to claim 4 characterised in that the edge (2) of the shaped portion (3) has notches (11) for receiving the loop portions (10).

6. A flat electrode according to claim 1 characterised in that the film hinges (6, 8) are disposed between the clamping plate (7) and the shaped portion (3) as well as between the shaped portion (3) and the cover plate (9).

7. A flat electrode according to claim 6 characterised in that the firm hinges (6, 8) extend in the longitudinal direction of the tongue.

8. A flat electrode according to claim 1 characterised in that the shaped portion (3) is connected both to the cover plate (9) and to the clamping plate (7) by film hinges (6, 8) extending in the longitudinal direction of the tongue (1), wherein the shaped portion (3) has a notch (26) extending perpendicularly to the film hinges (6, 8), for folding of the shaped portion (3).

9. A flat electrode according to one of claims 1 to 8 characterised in that the respectively outwardly disposed parts of the stack consisting of the shaped portion (3), the clamping plate (7) and the cover plate (9) are connected by a snap connection.

## Revendications

1. Electrode flexible en nappe, pour l'arrivée et le départ de courants vers ou depuis la peau d'un corps humain, sous la forme d'un support isolant (30) qui est recouvert d'au moins une feuille (31-33) conduisant l'électricité, laquelle se prolonge en une languette (1) dépassant de l'électrode en nappe, la languette (1) étant repliée de telle manière autour d'une partie moulée (3) en forme de plaque rigidifiante que les feuilles (31-33) conduisant l'électricité sont tournées vers l'extérieur, caractérisée en ce que
- l'extrémité de la languette (1) est maintenue entre la pièce moulée (3) et une plaque de serrage (7),
- les feuilles (31-33) conduisant l'électricité (31-33) sont libres sur le bord de la pièce moulée (3) pour la mise en contact,
- la partie de la languette (1) qui est située entre le bord frontal (2) de la pièce moulée (3) et l'électrode en forme de feuille à proprement parler, est recouverte par une plaque de couverture (9), et
- en ce que les parties pièce moulée (3), plaque de serrage (7) et plaque de couverture (9) sont reliées par des charnières à film.

2. Electrode en nappe selon la revendication 1, caractérisée en ce que les charnières à film sont placées entre pièce moulée (3) et plaque de serrage (7), ainsi qu'entre plaque de serrage (7) et plaque de couverture (9).

3. Electrode en nappe selon la revendication 2, caractérisée en ce que les charnières à film s'étendant transversalement à la languette (1).

4. Electrode en nappe selon la revendication 2 ou 3, caractérisée en ce qu'entre la plaque de serrage (7) et la plaque de couverture (9), des parties arquées (10) s'étendent dans la direction longitudinale de la languette (1), lesquelles présentent des charnières à film reliant la plaque de serrage (7) à la plaque de couverture (9).

5. Electrode en nappe selon la revendication 4, caractérisée en ce que le bord (2) de la pièce moulée (3) présente des encoches (11) destinées à recevoir les parties arquées (10).

6. Electrode en nappe selon la revendication 1, caractérisée en ce que les charnières à film (6, 8) sont placées entre la plaque de serrage (7) et la pièce moulée (3), ainsi qu'entre la pièce moulée (3) et la plaque de couverture (9).

7. Electrode en nappe selon la revendication 6, caractérisée en ce que les charnières à film (6, 8) s'étendent dans la direction longitudinale de la languette.

8. Electrode en nappe selon la revendication 1, caractérisée en ce que la pièce moulée (3) est reliée par des charnières à film (6, 8) s'étendant dans la direction longitudinale de la languette (1), aussi bien à la plaque de couverture (9) qu'à la plaque de serrage (7), la pièce moulée (3) présentant une encoche s'étendant perpendiculairement aux charnières à film (6, 8) pour plier la pièce moulée (3).

9. Electrode en nappe selon l'une quelconque des revendications 1 à 8, caractérisé en ce que les parties respectivement à l'extérieur de la pile constituée de la pièce moulée (3), de la plaque de serrage (7) et de la plaque de couverture (9) sont reliées par un assemblage à encliquetage.
